# EUROPEAN PATENT APPLICATION

(11) **EP 1 211 627 A1**
(43) Date of publication of application: **05.06.2002**
(21) Application number: 00811026.4
(22) Date of filing: 03.11.2000
(51) Int. Cl.: G06F 19/00

(54) **Method and system for registration, identifying and processing of drug specific data**

(71) Applicant: TheraSTrat AG, 4123 Allschwill (CH)
(72) Inventor: Gut, Joseph, Prof. Dr., 4203 Grellingen (CH); Bagatto, Dario, 4123 Allschwil (CH)
(74) Representative: Schwander, Kuno

(57) **Abstract**

The invention relates to a method for registration, identifying and processing of drug specific data and for making drugs available to individual patients without severe adverse drug reactions. The invention relates also to a system for carrying out such a method. According to the present invention, the system comprises a master database correlating patterns of gene expression and genetic polymorphisms with drug-induced i. e. drug-related adverse effects and drug structure. The system also comprises a predictive data-tool. In this tool the structural and genetic fingerprints predictive for adverse effects in individual patients due to treatment with a selected drug are stored. The method according the invention is characterised in that the master database being coupled to the database of the predictive data-tool in such a way that a user of the system can develop and carry out different screening approaches either to verify the sociability of drugs for a specific selected category of patients or to search a specific drug for a selected category of patients which do not have adverse drug reactions or to make risk-analyses.

## Description

The invention relates to a method for registration, identifying and processing of drug specific data and for making drugs available to individual patients avoiding adverse drug reactions (ADR). The invention relates also to a system for carrying out such a method.

Current methods and systems being used for access control of drug delivery are not sufficiently reliable and secure for the routine medical treatment of patients with drugs.

Thus, more than 2 million adverse drug reactions occur annually in the United States. An analysis of 39 prospective studies (JAMA 1998, 279, 1200-1205) shows that in 1994 2'216'000 patients have been hospitalised with adverse drug reactions, 106'000 patients of them with fatal outcome.

Recent examples of drugs affected by severe ADR include the following:

| Drug | Reaction |
|---|---|
| Troglitazone Rezulin | Anti-diabetes type II drug; severe liver toxicity, unexpected deaths; Warning labelling introduced by FDA; withdrawn from market. |
| Trovafloxacin Trovan | Antibiotic drug; unexpected severe liver toxicity with deaths occurring; |
| | call for ban of product that made US$ 68 Mio in its first year in the USA alone; boxed warning introduced by FDA. |
| Tolcapone Tasmar | Anti-Parkinson drug; severe liver toxicity with deaths; banned in UK, severely restricted in its use the in US. |
| Lazabemide Tempium | Anti-Alzheimer disease drug; severe liver toxicity in Phase III clinical trials; development aborted. |
| Sparfloxacin Zagam | Antibiotic drug; severe phototoxicity and cardiotoxicity; in EU limited to use in pneumonia. |
| Grepafloxacin Raxar | Antibiotic drug; severe cardiovascular events, several deaths; withdrawn from market. |
| Moxifloxacin Avelox | Antibiotic drug; FDA panel split about concerns on product safety; potential for prolonged QT-interval; approved. |

The challenge of the invention is to develop a new system usable for a controlled drug development and personalised drug delivery.

It is the object of the invention to develop a new sophisticated postgenomic knowledge management system which links chemical molecular modelling, bioinformatic, genetic, epidemiology, and molecular diagnostic data in order to develop prospective theragenomic information which allows the safe use of a given drug or a safe drug therapy, which is free of severe adverse drug reactions in each individual patient.

This object is achieved by a system and a method having the features of Patent Claim 1 and 6.

Advantageous embodiments of the invention form the subject of the dependent Claims.

It is generally known that the development of most drug-related adverse effects in man is based on the genetic and epigenetic predisposition, i.e. susceptibility of each individual. This predisposition is reflected in predictive patterns of structural properties, genetic polymorphisms and gene expression profiles that correlate with the development of drug related adverse effects.

The genetic predisposition is phenotypically revealed only when the individual carrier is exposed to (the) offending agent(s) or structural mimics thereof.

According to the present invention, the system comprises a master database correlating patterns of gene expression and genetic polymorphisms with drug-induced i. e. drug-related adverse effects and drug structure. This database stored on a separate server is preferably in a form such that data records of the following type can be entered:
- Basic drug information as for example information on intermediates, metabolites, adducts, targets, mimics, pathways, 2D-structures, 3D-structures and similarities.
- Clinical endpoint information as for example on offending drug, on type of endpoint, and on frequency.
- Drug-induced effects by genes as for example on receptors, promoters, transcription factors, responsive elements, expression pattern, gene-function, 3D-structure, adduct target and autoantigens.
- Drug-induced effects by allelic variants, such as, for example, SNP's, splice variants, and gene amplifications, on function(s), 3D-structure, frequencies, ethnic differences, predictive power, selectivity and sensitivity.

In accordance with the invention the system also comprises a predictive data-tool in electronic from. In this tool the structural and genetic fingerprints predictive for given adverse effects in individual patients due to treatment with a selected drug are stored.

Each individual patient-specific data in reality comprises a set of selected yet predictive structural and genetic information that can be presented on a gene chip to be used in theratyping of individual patients.

This means that a given drug or a given combination of drugs used as therapy regimen have its corresponding unique set of patient-specific data. These data can be further classified into various subgroups as for example in subgroups dependent of the sex and/or of the age of the patients or in subgroups corresponding to clinical endpoints and/or risk groups.

The method according the invention is characterised in that the master database is being coupled to the database of the predictive data-tool in such a way that a user of the system can develop different screening approaches either to verify the sociability of drugs for a specific selected category of patients or to search a specific drug for a selected category of patients which do not have adverse drug reactions or to make risk-analyses.

Therefore, it is also part of the invention that with an adequate tool available, theragenomics, can entirely be done electronically.

It is another part of the invention to make available the system, i.e. the basic database and the predictive-tool database to the user via the Internet. For that purpose the system further comprises means which allows the user to follow up their screening procedures in the databases via an Internet server, from where they can be called up merely by means of a login and a password.

The database type on which the method and system according to the invention is based is freely selectable and, for example, VISUAL FOXPRO. The same also applies to the computer operating system on which the database is based, which may be, for example, WINDOWS NT.

The invention will become apparent from the study of the following specification with reference to the attached drawing. In the drawing,

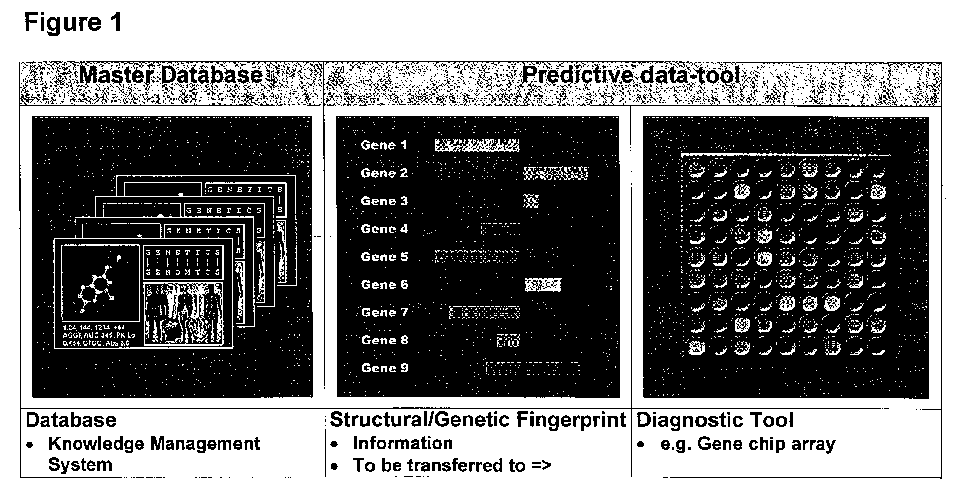

Figure 1 illustrates the principle of the correlation of drug-dependent data stored in the master database and in the predictive data-tool, and

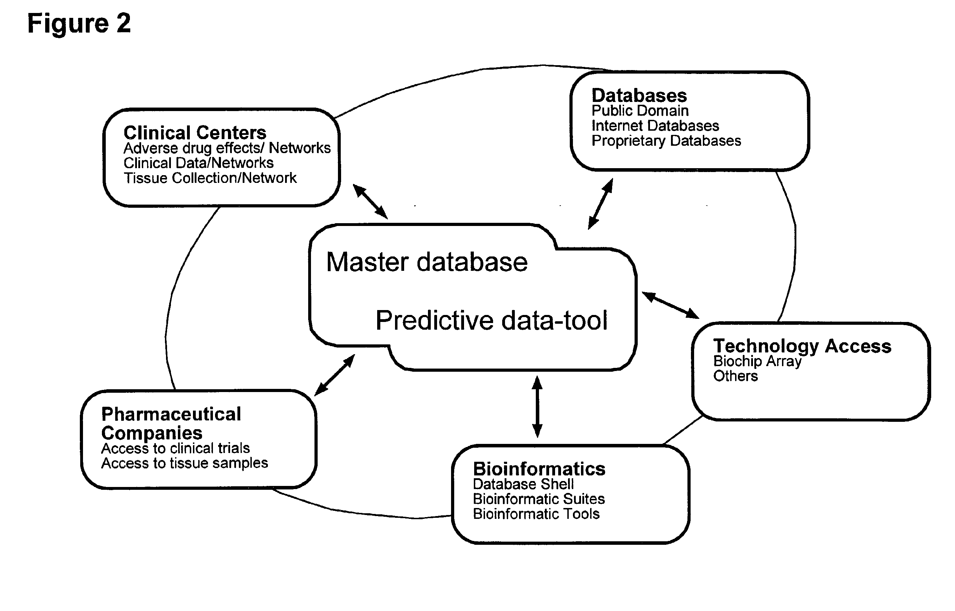

Figure 2 shows a diagram illustrating the principle of the function of the system and method according to the invention.

The three main features of the invention are:

| | |
|---|---|
| The master database | Which is |
| | - an object-oriented database-system, |
| | - based on a knowledge management-system, |
| | - Internet-based. |
| The predictive data-tool(s) | Which comprises |
| | - structural and genetic fingerprints (information), transferable to |
| | - diagnostic tools (for example, gene chip arrays). |
| The custom services | Which is characterised by consulting |
| | - pharmaceutical industries, |
| | - regulatory agencies (BAG, IKS, BPharm, EMEA, FDA), |
| | - societies (SOT, EUROTOX, others), |
| | - patients, physicians, health care providers, |
| | - financial analysts, investors, lawyers, courts and |
| | - The public. |

The master database correlates patterns of gene expression and genetic polymorphisms with drug-induced adverse effects and drug structure. This database can comprise drug specific date as follows:

| | |
|---|---|
| Structural Chemistry and Genomic | e.g., 2D-and 3D-structures; effects to receptors; ligands; metabolites; intermediates; function and structure of wt-gene and allelic variants gene products; chemical and postranslational modifications; others. |
| Pharmacogenetics | Mutated or re-arranged genes: e.g., CYP's; NAT-1; NAT-2; GST-_; p53; Rb: WT1; BRCA1; BRCA2; VHL; APC; NF-1;NF-2; MYS-1; splice variants (i.e., CD44v5, CD44v6); transcription factors; responsive elements; many others. |
| Expression genetics | Over- or under-expression of unmutated or mutated genes: e.g., CYP's; p15; p 107; p300; cyclin D1 (amplification); class II cancer genes (altered in expression); regulated transcription factors; many others. |
| Proteomics | Factors such as, for example, up- or down-regulation of protein expression; posttranslational modifications; chemical adduct formation; gain of new properties through modification; others. |
| Epigenetic networks and Environmental factors | Other factors, such as, for example DNA-methylation; signalling cascades; narangenin; bergotamine, alkaloids; retinoids; quinines; co-medications; man-made chemicals in the environment; infections; viral loads; status or type of disease. |

According to the invention, the input of data into the master database is effected by data-transfer from clinical centers, i.e from clinical data networks and/or from public domain, Internet and proprietor databases and/or from pharmaceutical companies and/or from bioinformatic databases. The data-transfer can ensure upon copying the data from diskettes, CD-ROM or DVD. Of course, it is also possible to transfer the data via the Internet.

The same procedure is effective for the set up of the predictive-data-tool.

The method according the invention couples the master database and the database of the predictive data-tool in such a way that a user of the system can develop different screening approaches either to verify the sociability of drugs for a specific selected category of patients or to search a specific drug, which show no adverse drug reactions for a selected category of patients, or to make risk-analyses.

Screening approaches can follow up according to the table below:

| Clinical Endpoint(s), such as, for example: | Approaches, such as, for example: |
|---|---|
| • Hepatitis C: Response to Therapy | Pharmacogenetics |
| | - Pattern of genetic polymorphism |
| • Transplantation : Chronic rejection | |
| | - Pattern of gene expression |
| • Ulcerogenesis of NSAID's COX-1 / 2 | Biostatistics |
| | - Predictive Power |
| • Haemolytic Anaemia | - Sensitivity |
| • Cholestasis | - Selectivity |
| • Hepatitis | - Pattern Frequency |
| • Thrombocytopenia | - (Allele) Frequency |
| • Agranulocytosis | - Level of confidence |
| | Molecular Modelling |
| | - Chemical structure |
| | - Structural similarity |
| | - Molecular mimicry |
| | - Homology modelling |

As mentioned above it is also a part of the invention to make available the system, i.e. the basic database and the predictive-tool database, to the public via the Internet. Preferred users are pharmaceutical industries, regulatory agencies, societies, patients, physicians and health care providers. Large pharmaceutical industries can use the system for postmarketing as well as for late and early drug-development purpose. Small pharmaceutical industries can use the system for compound selection and very early drug-development purpose.

The system and method according to the invention has enormous advantages. Thus, the pharmaceutical industries can screen their new products for adverse drug reactions in a simple and secure manner. Moreover, the data of the two databases of the system can be relatively simply updated and can be offered to various users on a data medium or by Internet. All in all, the method according to the invention also simplifies the search tasks of the industries to find out possible adverse reactions of drugs in development, so that it can, with high probability, be avoided that a drug has do be withdrawn from the market because of adverse drug reactions after coming onto the market.

## Claims

1. A sophisticated postgenomic system for registration, identifying and processing of drug specific data, **characterised in that** it links chemical, molecular modelling, bioinformatic, genetic, epidemiological, and molecular diagnostic data in order to develop prospective theragenomic information, and that it comprises
- a master database correlating patterns of gene expression and genetic polymorphisms with drug-induced i. e. drug-related adverse effects and drug structure,
- a data-tool for structural and genetic fingerprints predictive for adverse effects in individual patients, and
- means for coupling the master database and the predictive tool in such a way that electronically prospective theragenomic information can be developed which allows the safe use of a given drug or a safe drug therapy, which is, with high probability, free of adverse drug reactions in an individual patient.

2. System according to claim 1, **characterised in that** the master database is in a form such that data records of the following type can be entered:
- Basic drug information such as for example information to intermediates, metabolites, adducts, targets, mimics, pathways, 2D-structures, 3D-structures and similarities.
- Clinical endpoint information such as for example drugs, type of endpoint, frequency.
- Drug-induced effects by genes on, as for example, receptors, promoters, transcription factors, responsive elements, expression patterns, gene function, 3D-structure, adduct targets, and autoantigens.
- Drug-induced effects to allelic variants, such as for example, SNP's, splice variants, and amplifications on function(s), 3D-structure, frequencies, ethnic differences, predictive power, selectivity and sensitivity.

3. System according to claim 1 or 2, **characterised in that** the predictive tool is in a form such that each individual patient-specific data comprises a set of selected yet predictive structural and genetic information which are presented on, for example, a gene chip.

4. System according to claim 3, **characterised in that** the data are classified into various subgroups as for example in subgroups dependent of the sex and/or of the age of the patients or in subgroups corresponding to clinical pictures and/or risk groups.

5. System according to one of the claims 1 to 4, **characterised in that** it further comprises means which make available the master database and the predictive-tool to the public via the Internet.

6. A Method carrying out with a system according to one of the claims 1 to 5, **characterised in that** the master database is being coupled to the database of the predictive data-tool in such a way that a user of the system can develop and carry out different screening approaches either to verify the sociability of drugs for a specific selected category of patients or to search a specific drug for a selected category of patients which do not have adverse drug reactions or to make risk-analyses.

7. Method according to claim 6, **characterised in that** the screening approaches is realised online via the Internet.
